**Europäisches Patentamt**

**European Patent Office**

**Office européen des brevets**

(19)

(11) Veröffentlichungsnummer : **0 324 464 B1**

(12) # EUROPÄISCHE PATENTSCHRIFT

(45) Veröffentlichungstag der Patentschrift :
**15.04.92 Patentblatt 92/16**

(51) Int. Cl.$^5$ : **A61K 37/02**

(21) Anmeldenummer : **89100458.2**

(22) Anmeldetag : **12.01.89**

(54) **Verwendung von TNF und LT zur Herstellung von Arzneimitteln.**

(30) Priorität : **15.01.88 DE 3801026**
**31.10.88 DE 3837012**

(43) Veröffentlichungstag der Anmeldung :
**19.07.89 Patentblatt 89/29**

(45) Bekanntmachung des Hinweises auf die
Patenterteilung :
**15.04.92 Patentblatt 92/16**

(84) Benannte Vertragsstaaten :
**AT BE CH DE ES FR GB IT LI LU NL SE**

(56) Entgegenhaltungen :
**CHEMICAL ABSTRACTS, Band 107, Nr. 21, 23.
November 1987, Seite 213, Zusammenfassung
Nr. 192301w, Columbus, Ohio, US; & JP-A-61
280 436 (DAINIPPON PHARMACEUTICAL CO.,
LTD) 11-12-1986
GOODMAND AND GILMAN'S "THE PHARMA-
COLOGICAL BASIS OF THERAPEUTICS", 7.
Edition, 1985, Seiten 904-905, MacMillan Publishing Co., New York, US
CIBA FOUNDAT. SYMP., Band 131, 1987, Seiten 206-227; D.R. SPRIGGS et al.: "Clinical
studies with tumour necrosis factor"
Razis et al.: Diuretics in malignant effusions
and edemas of generalized cancer. Journal of
Medicine 7, No. 6, 1976
Schäfer E.: Behandlung des malignen Aszites.
DMW 113, Jg. Nr. 7, 264 - 267, 1988
Niederle et al.: Phase I study of rec. human
TNF alpha in advanced malignant disease.
Cancer Immunol. Immunother. 29, 144-150,
1989
Wiedemann et al.: Phase I trial of i.v. continuous infusions of tumor necrosis factor in
advanced metastatic carcinomas. J. of Cancer
Res. Clin. Oncol. 115, 189-192, 1989
Creaven et al.: Phase I clinical trial of recombinant human tumor necrosis factor. Cancer
Chemother. Pharmacol. 20, 137-144, 1987**

(56) Entgegenhaltungen :
**Sherman et al.: Recombinant human tumor
necrosis factor administered as a five day
continuous infusion in cancer patients: Phase
I toxicity and effects on lipid metabolism. J. of
Clin. Oncol. 6, No. 2, 344-350, 1988
Jakubowski et al.: Phase I trial of i.m. administered tumor necrosis factor in patients with
advanced cancer. J. of Clin. Oncol. 7, No. 3,
298-303, 1989
Kruger et al.: A phase I trial of recombinant
human TNF alpha administered by continuous
infusion for 120 hours in patients with advanced cancer (meeting abstract). Proc. Annu.
Meet. Am. Soc. Clin. Oncol. 7, A 657, 1988**

(73) Patentinhaber : **KNOLL AG
Knollstrasse 32
W-6700 Ludwigshafen (DE)**

(72) Erfinder : **Schlick, Erich, Dr.
Reiherstrasse 9
W-6701 Otterstadt (DE)**
Erfinder : **Kaufmann, Manfred, Dr.
Neuer Weg 16
W-6900 Heidelberg (DE)**
Erfinder : **Raeth, Ulrich, Dr.
Schroederstrasse 5
W-6900 Heidelberg (DE)**

(74) Vertreter : **Karau, Wolfgang, Dr. et al
BASF Aktiengesellschaft Carl-Bosch-Strasse
38
W-6700 Ludwigshafen (DE)**

## Beschreibung

TNF (Tumor-Nekrose-Faktor) ist eine Tumor-zerstörende Substanz, die zur Bekämpfung maligner Tumoren Verwendung finden soll. Die Struktur dieser Substanz ist in Nature 312, 724 (1984) beschrieben worden. LT (Lymphotoxin) ist eine dem TNF verwandte Substanz und besitzt eine ähnliche Wirkung (Nature 312, 721 (1984)). Entsprechendes gilt für Muteine des TNF (EP 168 214, EP 205.038, EP 251 037, WO 86(0231)) und des LT (EP 164 965, DE 3 620 656).

Es wurde nun gefunden, daß sich diese Substanzen auch zur Prophylaxe und Therapie von Ergüssen in Körpenhöhlen eignen.

Gegenstand der Erfindung ist die Verwendung von TNF, LT und deren Muteinen zur Herstellung von Arzneimitteln zur Prophylaxe und Therapie von Ergüssen in Körperhöhlen bzw. die Verwendung von TNF zur Prophylaxe und Therapie von Ergüssen in Körperhöhlen.

Als Ergüsse sind Pleuraergüsse, Pericardergüsse, Peritonealergüsse (Ascites) und Gelenkergüsse zu verstehen, die als Folge maligner und nicht-maligner Grunderkrankungen auftreten können. Beispiele für maligne Ergüsse sind insbesondere Ascites im Gefolge von Ovarialtumoren, gastro-intestinalen Tumoren, Hypernephromen und Mammacarcinomen sowie Pleuraergüsse im Gefolge von Bronchialcarcinomen, Mesotheliomen, Mammarcarcinomen und Hypernephromen. Als Beispiele für nicht-maligne Ergüsse sind vor allem zu nennen: Ascites im Gefolge von Lebererkrankungen, wie z.B. Leberzirrhose, Virus-bedingte Pleura- und Pericardergüsse sowie Gelenkergüsse bei entzündlichen und/oder degenerativen Gelenkerkrankungen.

Da TNF, LT und deren Muteine Proteine darstellen, welche im Magen-Darm-Trakt zerstört werden, können sie nur parenteral, vorzugsweise intravenös, intraperitoneal, intrapleural und intraarticulär verabfolgt werden. Hierzu eignen sich sterile isotonische Lösungen. Diese lassen sich beispielsweise herstellen, indem man das Protein in einer blutisotonischen wäßrigen Lösung löst, die Lösung steril filtriert und in Ampullen abfüllt. Der pH-Wert der Lösung liegt vorzugsweise zwischen 5 und 8, insbesondere bei etwa 7,5.

Die zu applizierende Dosis liegt pro Patient bei 10 bis 1000 µg, vorzugsweise 30 bis 700 µg Protein pro $m^2$ Körperoberfläche. Diese Dosis wird 1- bis 2-mal wöchentlich für eine Behandlungsdauer von 1 bis 6 Wochen verabreicht. Der Behandlungszyklus kann bei Bedarf mehrfach wiederholt werden.

Die Wirksamkeit von TNF, LT und deren Muteinen ließ sich am Beispiel des TNF wie folgt zeigen:

12 Patienten mit malignem Ascites im Gefolge von Ovarialcarcinomen bzw. 10 Patienten mit malignem Ascites im Gefolge von gastrointestinalen Tumoren erhielten 1-mal wöchentlich eine intraperitoneale Infusion von TNF in Dosen von 20 bis 200 µg pro $m^2$ Körperoberfläche über einen Zeitraum von 1 bis 6 Wochen. Bei keinem der 12 Patienten mit Ovarialcarcinomen konnte nach der Behandlung maligner Ascites nachgewiesen werden. Von den 10 behandelten Patienten mit gastrointestinalen Tumoren zeigten 7 nach der Behandlung keinen Ascites mehr.

Herstellung einer Applikationsform

100 mg TNF werden in 300 ml 20 mM Natriumphosphatpuffer pH 7,5 gelöst. Die Lösung wird mit Kochsalz blutisotonisch eingestellt. Nach Zugabe von 1 g humanem Serumalbumin wird über einen Porenfilter (Porengröße 0,1 bis 0,2 µm) steril filtriert. Jeweils 3 ml werden steril in Ampullen gefüllt.

Die so erhaltenen Ampullen können direkt injiziert oder nach einer weiteren Verdünnung durch Zugabe zu einer blutisotonischen Lösung, die 0,25 % humanes Serumalbumin enthält, infundiert werden.

## Patentansprüche

1. Verwendung von TNF, LT und deren Muteinen zur Herstellung von Arzneimitteln zur Prophylaxe und Therapie von Ergüssen in Körperhöhlen.

2. Verwendung von TNF zur Herstellung von Arzneimitteln zu Prophylaxe und Therapie von Ergüssen in Körperhöhlen.

## Claims

1. Use of TNF, LT or a mutein thereof for preparing drugs for the prophylaxis and therapy of effusions in body cavities.

2. Use of TNF for preparing drugs for the prophylaxis and therapy of effusions in body cavities.

**Revendications**

1. Utilisation du TNF, de la LT et de leurs mutéines pour la préparation de médicaments pour la prophylaxie et la thérapie des épanchements dans les cavités de l'organisme.

2. Utilisation du TNF pour la préparation de médicaments pour la prophylaxie et la thérapie des épanchements dans les cavités de l'organisme.